# EUROPEAN PATENT SPECIFICATION

(11) **EP 4 135 814 B1**
(45) Date of publication and mention of the grant of the patent: **06.08.2025**
(21) Application number: 20720020.5
(22) Date of filing: 16.04.2020
(51) Int. Cl.: A61M 16/08, A61M 16/12, A61M 16/00

(54) **DEVICE AND SYSTEM FOR RESPIRATORY SUPPORT**
VORRICHTUNG UND SYSTEM ZUR ATEMUNTERSTÜTZUNG
DISPOSITIF ET SYSTÈME D'ASSISTANCE RESPIRATOIRE

(43) Date of publication of application: 22.02.2023
(73) Proprietor: Neores AB, 831 45 Östersund (SE)
(72) Inventor: DREVHAMMAR, Thomas, 831 43 Östersund (SE); NILSSON, Kjell, 831 45 Östersund (SE)
(74) Representative: AWA Sweden AB
(86) International application number: PCT/EP2020/060780
(87) International publication number: WO 2021/209142

(56) References cited:
- EP-A1- 0 512 285
- EP-A1- 2 231 244
- WO-A1-2012/108826
- WO-A1-2013/067164
- US-A1- 2009 250 059
- US-A1- 2009 253 995
- US-B1- 6 915 705

## Description

### FIELD OF THE INVENTION

The present disclosure relates to a device and a system for positive pressure ventilation (PPV) and continuous positive airway pressure (CPAP) treatment for e.g. neonatal resuscitation and initial respiratory support.

### BACKGROUND OF THE INVENTION

Positive pressure ventilation (PPV) and continuous positive airway pressure (CPAP) are two types of mechanical respiratory support commonly used for resuscitation and stabilisation of newborn infants. Which of the two respiratory support systems to use depends on whether the infant is breathing or not. While PPV is used for the non-breathing infant, CPAP is used for the breathing infant. An infant that is not breathing after birth should be ventilated. This can be accomplished with PPV using e.g. a face mask, or an endotracheal tube. In the majority of cases a mask is used. If the infant starts to breathe or was breathing after birth, support of ventilation using CPAP is the recommended treatment for several conditions. After return of spontaneous breathing some infants will still need PPV intermittently if they do not breathe adequately or stop breathing. This is particularly common when treating premature neonates. Thus, both types of support are common and often the need changes during the resuscitation period. During both PPV and CPAP the distending pressure helps the lung to maintain aeration. This is referred to as positive end expiratory pressure (PEEP) when providing PPV and is, for this type of device, functionally equivalent to CPAP.

A device and system which can provide both positive pressure ventilation and continuous positive airway pressure is disclosed in WO2012/108826. The device comprises a first fresh gas flow tube arranged to provide a first fresh gas flow, a second fresh gas flow tube, a variable flow CPAP generator, a connector connectable to any infant interface, and a pressure release valve. The variable flow CPAP generator comprises a first connection portion to which the first fresh gas flow tube is connected, a second connection portion to which the infant interface connector is connected, and a third connection portion which is an outlet of the variable flow CPAP generator having an open end. The second fresh gas flow tube bypasses the variable CPAP generator and is arranged to provide a second fresh gas flow which is added to the first fresh gas flow in the positive pressure ventilation mode when the open end of the outlet is occluded. Thereby, in the PPV mode, the pressure is increased at a higher rate than if only the first fresh gas flow had been used, which allows for a shortened inspiratory rise time. The system can be used for non-invasive ventilation and provides a low imposed work of breathing for the breathing child treated with CPAP and an easy switch between PPV and CPAP respiratory support without change of equipment.

For an optimal functioning of the system it is necessary to adjust the fresh gas flow supplied during resuscitation through the use of the second fresh gas flow. Adding the second fresh gas flow in the PPV mode provides an adjustable rise time, which e.g. can be shortened by increasing the second fresh gas flow. This is desirable to provide a stable and efficient ventilation. However, applying the second fresh gas flow is not possible in all medical installations, due to e.g. lack of outputs at the gas source for connecting the second fresh gas flow tube. Further, the second fresh gas flow increases the complexity of the device and may increase the risk of user errors with potentially serious consequences. Consequently, making use of a second fresh gas flow through the second fresh gas flow tube is not always possible resulting in a less efficient resuscitation treatment.

Therefore, there is a need within the technical field of devices providing both PPV and CPAP to overcome the problems that exist today.

### SUMMARY OF THE INVENTION

The invention is defined in the appended claims. It is an object of the present disclosure to provide a device for PPV and CPAP treatment that alleviates the above-mentioned problems. The object is achieved by a device for PPV and CPAP treatment according to the present disclosure as disclosed herein.

Thus, in accordance with an aspect of the present disclosure there is provided a device for positive pressure ventilation and continuous positive airway pressure treatment comprising:
- a fresh gas flow inlet arranged to receive a fresh gas flow from a fresh gas flow tube connectable thereto;
- a patient interface end which is connectable with a patient interface;
- an outlet having an open end; and
- several variable flow CPAP generators, wherein each of the several variable flow CPAP generators is connected with the fresh gas flow inlet, the patient interface end and the outlet of the device, wherein each of the variable flow CPAP generators comprises a first connection portion connected with the fresh gas flow inlet, a second connection portion connected with the patient interface end, and a third connection portion connected with the outlet of the device.

This provides a simplified device for CPAP and PPV treatment, particularly intended for resuscitation and stabilisation of infants. The device is easy to use and allows rapid switch between PPV and CPAP without change of equipment. A safe and quick switch between these types of respiratory support will improve care for an unstable child and lead to higher quality in resuscitation with reduced mortality and morbidity.

Providing several variable flow CPAP generators, each connected with the fresh gas flow inlet, the patient interface end and the outlet of the device, allows operating at a higher drive flow, i.e. with a higher fresh gas flow provided to the fresh gas flow inlet of the device, while maintaining the same stable pressure at the patient interface end in the CPAP mode, as compared to when providing one sole variable flow CPAP generator. Further, in the PPV mode, when the outlet of the device is occluded, the higher fresh gas flow used for the device will be delivered to the patient, resulting in a rapid increase of the pressure and generating an inflation breath.

The provision of a fresh gas flow inlet simplifies gas supply to the device, since one sole driver flow of fresh gas is needed for the device, i.e. for the one sole fresh gas flow inlet. This further allows for a reduction in size of the device with respect to when two driver flows are required, thereby also facilitating the handling and use of the same. A further advantage with providing only one driver flow for the device as herein disclosed is that it allows integration of the device in both low tech and high-tech ventilator equipment, requiring only one gas output channel for fresh gas supply from the ventilator equipment. That is, the device can be installed in a system comprising just one gas output channel for fresh gas supply. This allows integration of the device with existing CPAP drivers and ventilators without any modification of the hardware. Additionally, another advantage is that providing humidification of the flow to the fresh gas flow inlet is simple, as opposed to for a device comprising two driver flows.

The driver flow, i.e. the fresh gas flow supplied to the fresh gas flow inlet, can be set to an amount that allows for a desired CPAP to be generated by the variable flow CPAP generators. When the open end of the outlet of the device is occluded, corresponding to the PPV mode, the entire driver flow will reach the patient interface end and, further, the patient, resulting in an increased flow for the inflation breath. The driver flow being increased with respect to a device comprising only one variable flow CPAP generator providing the same level of CPAP in the CPAP mode provides for a decreased rise time of the pressure when the outlet of the device is occluded, which is advantageous.

Further, the device provides for a compact arrangement of the several variable flow CPAP generators, and a compact device. In operation, the fresh gas flow entering the fresh gas flow inlet of the device will further be divided between the several first connection portions of the several CPAP generators to flow therethrough. The second connection portions of the variable flow CPAP generators being connected with the patient interface end of the device provide a stable level of CPAP at the patient interface end when the device operates in the CPAP mode. Finally, providing the third connection portions of the several variable flow CPAP generators connected with the outlet of the device allows for a smooth operation of the device, as it can be set in the PPV mode simply by occluding the one outlet of the device. Further, providing variable flow CPAP generators with first, second, and third connection portions as previously described is advantageous since such variable flow CPAP generators generally provide a stable level of CPAP and low imposed work of breathing for the patient when used.

For purposes of this disclosure, the words "infant" and "child" are intended to encompass a patient such as a newborn, and a neonatal child which is in need of neonatal resuscitation and initial respiratory support. The words are also intended to encompass children and toddlers of up to around 10 kg of weight. The fresh gas flow is adapted to the weight and size of the child such that a desirable respiratory rate is attained. For purposes of this disclosure, the wording "fresh gas flow" is air, oxygen or a mixture of these that flows through the system and its parts, and the wording "fresh gas flow tube" is wherein the fresh gas flows.

For purposes of this disclosure, the wording "variable flow CPAP generator" is a device intended to encompass any continuous positive airway pressure device where the CPAP level is adjusted by varying the fresh gas flow.

For the purposes of this disclosure, the wording "driver flow" will be used as a synonym to the fresh gas flow provided to the fresh gas flow inlet of the device.

For purposes of this disclosure, the word "patient interface" is intended to encompass any interface that is suitable for connecting to a patient, e.g. an infant or child, such as a pair of nasal prongs, a mask, an endotracheal tube or any other suitable device.

There are different ways of arranging the device in order to achieve the advantages set forth above.

In accordance with an embodiment of the device, the device further comprises an inlet chamber arranged at the fresh gas flow inlet, a patient interface chamber arranged at the patient interface end, and an outlet chamber arranged at the outlet of the device, wherein each of the several variable flow CPAP generators is connected with the fresh gas flow inlet, the patient interface end, and the outlet through the inlet chamber, the patient interface chamber and the outlet chamber, respectively. That is, the inlet chamber, the patient interface chamber, and the outlet chamber are common to the several variable flow CPAP generators, as are the fresh gas flow inlet, the patient interface end, and the outlet of the device. This provides a compact device which is easy to handle.

In accordance with an embodiment of the device, the device comprises three variable flow CPAP generators, each of which comprises a first connection portion connected with the fresh gas flow inlet, a second connection portion connected with the patient interface end, and a third connection portion connected with the outlet of the device. In another embodiment of the device, the number of variable flow CPAP generators is four. Generally, any number of variable flow CPAP generators larger than one can be provided according to the inventive concept. The drive flow can then be adapted to the patient on which the device is to be used, in order to provide the desired CPAP in the CPAP mode. For smaller infants, such as neonates, a device comprising two variable flow CPAP generators may be sufficient to provide the desired rise time when operated in the PPV mode. For a larger child, e.g. having a weight of around 10 kg, it may be advantageous to use a device comprising three or four variable flow CPAP generators in order to achieve the desired rise time for that patient when operated in the ventilation mode. It will be understood from the present disclosure that the higher the number of variable flow CPAP generators in the device, the higher driver flow can be used, resulting in a faster pressure increase, i.e. shortened rise time, in the PPV mode. Preferably, the number of variable flow CPAP generators is between two and four. Providing between two and four variable flow CPAP generators does not necessarily contribute to a larger device for PPV and CPAP treatment as the variable flow CPAP generators could be fitted within the outer measures of devices known in the art. However, providing more than four variable flow CPAP generators is also conceivable within the concept of the present disclosure.

In accordance with an embodiment of the device, it further comprises a pressure release connection portion having a first end connected with the patient interface end of the device and a second end connectable with a pressure release tube. The pressure release connection portion is generally tubular and extends through a portion of the device between the patient interface end and a side wall of the device, forming an internal passage there through. A pressure release tube is connectable with the hole through the side wall provided by the second end of the pressure release connection portion.

According to another aspect of the present disclosure, there is provided a system for PPV and CPAP treatment comprising a device as disclosed herein, a fresh gas flow tube, a fresh gas source connected with the fresh gas flow inlet by means of the fresh gas flow tube, and a pressure release valve arranged to prevent an excessive positive pressure in a PPV mode. Thus, the system includes the fresh gas source and a pressure release valve which is set to a specific opening pressure adapted to suit the patient to be treated.

In accordance with an embodiment of the system, it is arranged such that when the open end of the outlet of the device is occluded, the pressure will increase from the variable flow CPAP generators until an opening pressure of the pressure release valve is reached, which increase in pressure results in an inspiratory flow, whereby the pressure in the system will remain at the set PPV pressure until the occlusion is removed from the outlet, and when the occluded outlet is opened, the pressure will return to the set CPAP level, whereby the reduction in pressure leads to an expiratory flow.

In accordance with an embodiment of the system, it is arranged such that during spontaneous breathing, the patient flow and the fresh gas flow leave the system through the variable flow CPAP generators keeping the positive pressure within the airway of the patient stable, by varying the flow that generates the CPAP, whereby the CPAP in the airway can be adjusted as needed.

In accordance with an embodiment of the system, the pressure release valve is connected with one of the patient interface end and the outlet of the device. These are two alternative ways of arranging the pressure release valve in the system, where the main aim is to arrange it in a position where the pressure of interest is measurable in a reliable way. Arranging the pressure release valve in connection with the outlet of the device allows for removal of a pressure release tube and a pressure release connection portion, providing a compact device and system.

In accordance with an embodiment of the system comprising a device having a pressure release connection portion, the pressure release valve is connected with the pressure release connection portion. In an embodiment of this system, it further comprises a pressure release tube at which the pressure release valve is arranged. This may be a more familiar way of arranging the pressure release valve for a user.

According to an embodiment of the system, it further comprises a pressure measuring device. Thereby, it is possible to easily monitor the operation of the system and make desirable adjustments of the fresh gas flow. Preferably, the pressure measuring device is arranged at the patient interface end of the device. In accordance with an embodiment of the system having a device comprising a pressure release connection portion, the pressure measuring device is connected with the patient interface end through the pressure release connection portion. Naturally, the main aim is to arrange the pressure measuring device in a position where the pressure of interest is measurable in a reliable and accurate way.

The different embodiments of the system can be summarized as a system comprising a device having a fresh gas flow inlet connected with several variable flow CPAP generators, in turn connected with a common patient interface end and an outlet of the device. The system further comprises a fresh gas flow tube connected at one end to the fresh gas flow inlet and at the other end to a fresh gas source for supplying a fresh gas flow to the device. A pressure release valve is further comprised in the system, being connected with one of the outlet and the patient interface end of the device. Optionally, the system comprises a pressure measuring device arranged at the patient interface end of the device for accurate and reliable measuring of the pressure thereat.

The device as disclosed herein can be used in PPV and CPAP treatment for neonatal resuscitation and initial respiratory support.

The system as disclosed herein can be used for PPV and CPAP treatment for neonatal resuscitation and initial respiratory support.

These and other aspects, and advantages of the disclosure will be apparent from and elucidated with reference to the embodiments described hereinafter.

### BRIEF DESCRIPTION OF THE DRAWINGS

The disclosure will now be described in more detail and with reference to the appended drawings. The drawings are included to provide a further understanding of the present disclosure and are incorporated in and are a part of this specification. Other embodiments of the present disclosure, and many of the intended advantages of the present disclosure, will be readily appreciated as they become better understood by reference to the following detailed description. The elements of the drawings are not necessarily to scale relative to each other. The same reference numerals designate corresponding similar parts.
Fig. 1 is a schematic, partly cut-away, illustration of an embodiment of the device according to the present disclosure;
Fig. 2 is a schematic, partly cut-away, illustration of another embodiment of the device according to the present disclosure;
Fig. 3 is a schematic cross-sectional illustration of yet another embodiment of the device according to the present disclosure;
Figs. 4-5 are schematic cross-sectional illustrations of embodiments of the system according to the present disclosure;
Figs. 6a-b schematically illustrate flows of the system shown in Fig. 4 during positive pressure ventilation, i.e. PPV mode; and
Fig. 7 schematically illustrates flows in the system shown in Fig. 4 during spontaneous ventilation, i.e. CPAP mode.

### DESCRIPTION OF PREFERRED EMBODIMENTS

Examples of different embodiments of the present disclosure are provided. Common for the embodiments of the device for PPV and CPAP treatment described herein is that they comprise a fresh gas flow inlet 2, a patient interface end 3, an outlet 4 having an open end 5, and several variable CPAP generators 6, each connected with the fresh gas flow inlet 2, the patient interface end 3, and the outlet 4. More particularly, the device further comprises an inlet chamber 21 at the fresh gas flow inlet 2, a patient interface chamber 31 at the patient interface end 3, and an outlet chamber 41 at the outlet 4. Each of the variable flow CPAP generators 6 is connected with the fresh gas flow inlet 2, the patient interface end 3, and the outlet 4 through the inlet chamber 21, the patient interface chamber 31, and the outlet chamber 41, respectively. That is, the inlet chamber 21, the patient interface chamber 31, and the outlet chamber 41 are common to the several variable flow CPAP generators 6, as are the fresh gas flow inlet 2, the patient interface end 3, and the outlet 4 of the device.

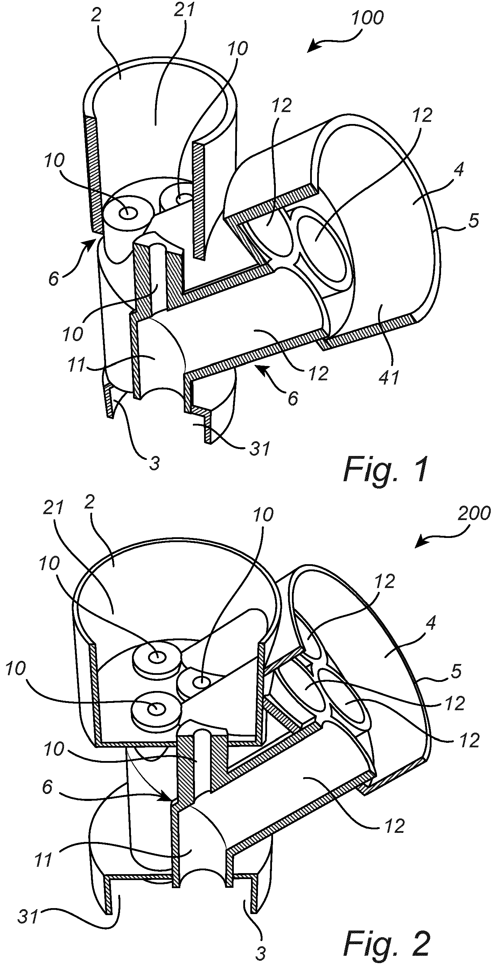

With reference to Fig. 1, according to a first embodiment of the device 100, the device 100 comprises three variable flow CPAP generators 6, arranged adjacent to each other. Each of the variable flow CPAP generators 6 comprises a first connection portion 10, a second connection portion 11, and a third connection portion 12. The first connection portions 10 of the variable flow CPAP generators 6 are connected with the fresh gas flow inlet 2, the second connection portions 11 are connected with the patient interface end 3, and the third connection portions 12 are connected with the outlet 4 of the device 100. More particularly, the first connection portions 10 of the variable flow CPAP generators 6 are connected with the fresh gas flow inlet 2 through the inlet chamber 21. The inlet chamber 21 is generally delimited by the inner walls of a tubular portion extending between the fresh flow gas inlet 2 and the first connection portions 10 of the variable flow CPAP generators 6. Correspondingly, the second connection portions 11 are connected with the patient interface end 3 through the patient interface chamber 31. The patient interface chamber 31 is generally defined by a tubular portion extending between the second connection portions 11 and the patient interface end 3. It may, however, be of any other shape suitable for the purpose of providing a common chamber connecting the second connection portions 11 of the variable flow CPAP generators 6 to the common patient interface end 3 of the device 100. The patient interface end 3 of the device 100 is further connectable with any patient interface, such as for example a face mask, nasal prongs, or an endotracheal tube.

In a corresponding manner, the third connection portions 12 of the variable flow CPAP generators 6 are connected with the outlet 4 of the device 100 through the outlet chamber 41. The outlet chamber 41 is typically tubular and arranged as a protruding tube ending, which extends between the second connection portions 12 and the outlet 4 having a free open end 5. The three variable flow CPAP generators 6 of the exemplifying embodiment shown in Fig. 1 are thus arranged in parallel, extending between the common fresh flow gas flow inlet 2, patient interface end 3 and outlet 4 of the device 100, providing a compact device 100.

In the exemplifying embodiment shown in Fig. 1, the second and third connection portions 11, 12 of each variable flow CPAP generator 6 are arranged at an angle to each other. The first connection portion 10 is connected with the third connection portion 12 at an angle, and is further arranged substantially coaxially with the second connection portion 11. Such a geometry of the variable flow CPAP generators 6 is advantageous as it provides a stable CPAP generated by the variable flow CPAP generators 6, and a low imposed work of breathing for the patient when in use. However, providing several variable flow CPAP generators 6 in the device 100 having a different geometry is also conceivable within the concept of the present disclosure.

With reference to Fig. 2, according to another embodiment of the device 200, the device 200 comprises four variable flow CPAP generators 6. Each of the four variable flow CPAP generators 6 comprises a first connection portion 10, a second connection portion 11, and a third connection portion 12. The first connection portions 10 of the variable flow CPAP generators 6 are connected with the fresh gas flow inlet 2, the second connection portions 11 are connected with the patient interface end 3, and the third connection portions 12 are connected with the outlet 4 of the device 200. Also in this embodiment, the first connection portions 10 of the variable flow CPAP generators 6 are connected with the fresh gas flow inlet 2 through the inlet chamber 21. Correspondingly, the second connection portions 11 are connected with the patient interface end 3 through the patient interface chamber 31, and the third connection portions 12 of the variable flow CPAP generators 6 are connected with the outlet 4 of the device 100 through the outlet chamber 41. The outlet chamber 41 is typically tubular and arranged as a protruding tube ending, which extends between the second connection portions 12 and the outlet 4 having a free open end 5. The patient interface end 3 of the device 100 is connectable with any patient interface, such as for example a face mask, nasal prongs, or an endotracheal tube. The four variable flow CPAP generators 6 are arranged in parallel, extending between the fresh flow gas flow inlet 2, the patient interface end 3 and the outlet of the device 4, providing a compact device 200.

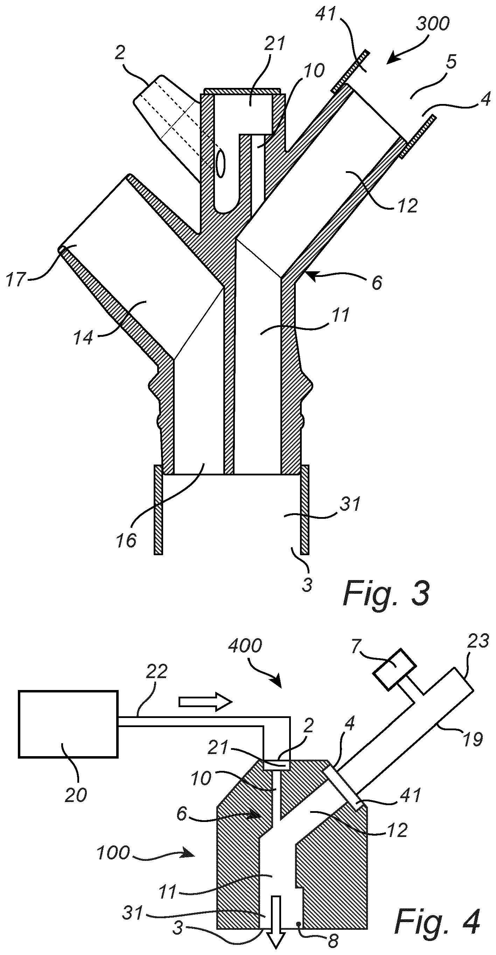

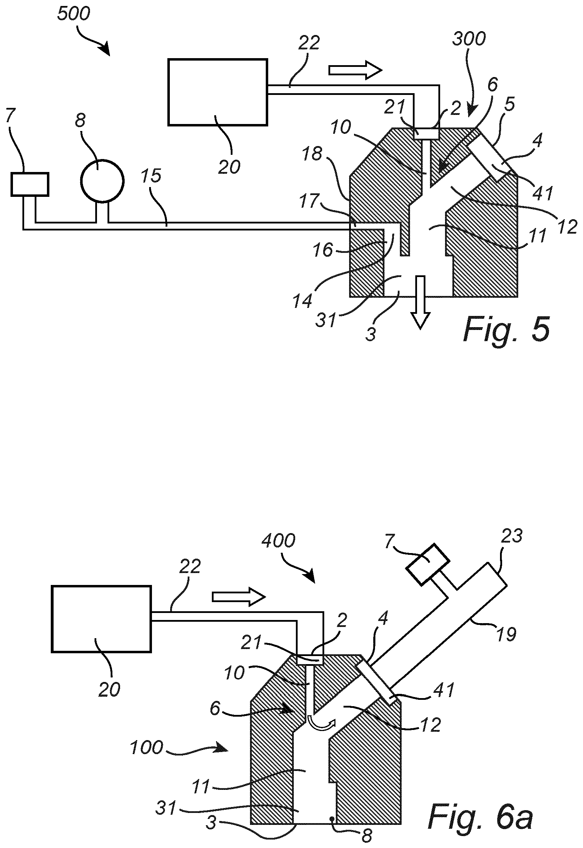

With reference now to Fig. 3, according to yet another embodiment, the device 300 comprises three variable flow CPAP generators 6 arranged as described with respect to Fig. 1. Due to the cross-sectional illustration of Fig. 3, only one of the variable flow CPAP generators 6 is visible therein. Furthermore, the device 300 comprises a pressure release connection portion 14 having a first end 16 and a second end 17. The first end 16 is connected with the patient interface end 3 of the device 300 and the second end 17 is connectable to a pressure release tube 15, see Fig. 5. More particularly, the first end 16 is connected with the patient interface end 3 through the patient interface chamber 31. The pressure release connection portion 14 is generally tubular and extends partially in parallel with the second connection portions 11 of the variable flow CPAP generators 6, and partially at an angle thereof towards a side wall 18 of the device, see Fig. 5. This embodiment is useful in a system that will be described with reference to Fig. 5. It should be noted that the pressure release connection portion 14 is applicable to a device having any number of variable flow CPAP generators.

A first embodiment of the system 400, as shown in Fig. 4, comprises the embodiment of the device 100 described with reference to Fig. 1. Due to the cross-sectional illustration shown in Fig. 4, only one of the three variable flow CPAP generators 6 is visible therein. The system 400 further comprises a fresh gas source 20, which provides a fresh gas flow to the fresh gas flow inlet 2 through a fresh gas flow tube 22 of the system 400. The level of CPAP is adjusted at the fresh gas source 20. The system further comprises a pressure release valve 7 and a pressure measuring device 8. The pressure release valve 7 is here arranged in connection with the outlet 4 of the device 100. More particularly, the system 400 further comprises an outlet tube 19 connected with the outlet 4 of the device 100. The pressure release valve 7 is arranged along the outlet tube 19. The outlet tube 19 comprises an open end 23. The pressure measuring device 8 is arranged at the patient interface end 3 of the device 100. The pressure measuring device 8 can be omitted in all embodiments, however, if there is no interest in measuring the pressure.

With reference to Fig. 5, according to a second embodiment of the system 500, the system 500 comprises the device 300 as described with reference to Fig. 3, i.e. the device comprising a pressure release connection portion 14. As for the embodiment shown in Fig. 4, due to the cross-sectional illustration of the system 500, only one of the variable flow CPAP generators 6 is visible in Fig. 5. The system 500 further comprises a pressure release tube 15 connected with the pressure release connection portion 14. The pressure release valve 7 is arranged at an end of the pressure release tube 15 which is distal to the pressure release connection portion 14. The pressure measuring device 8 is here arranged at the pressure release tube 15 between the pressure release valve 7 and the pressure release connection portion 14.

The system is operated as follows. Reference will be made to the first embodiment of the system 400, but the second embodiment of the system 500 has a corresponding operation. Oxygen concentration and fresh gas flow are adjusted by a standard blender and a flow meter. The fresh gas flow could be varied and is typically set to between 5 and 15 litres per minute. Typically, the fresh gas flow provided is between 10 and 12 litres per minute. A fresh gas flow in this range should prevent rebreathing, provide flow to achieve an adequate inspiration flow, volume and time, and provide some allowance for leakage at the patient interface. However, a fresh gas flow above 15 litres per minute is also possible to provide, particularly for treatment of an older child. The system is further configured such that this range of supplied fresh gas flow generates a level of CPAP of 3-10 cm H₂O. In a preferred embodiment, the generated level of CPAP is 5-6 cm H₂O.

The fresh gas flow is used to drive the variable flow CPAP generators 6. The fresh gas flow to the variable flow CPAP generators 6 is always adjustable. In the exemplifying embodiment of the device 100 comprising three variable flow CPAP generators 6, a typical value of the fresh gas flow provided to the fresh gas flow inlet 2 is 5-15 liters per minute, typically generating a CPAP level of 3-10 cm H₂O. To provide higher levels of CPAP, the flow driving the variable flow CPAP generators 6 can be increased further.

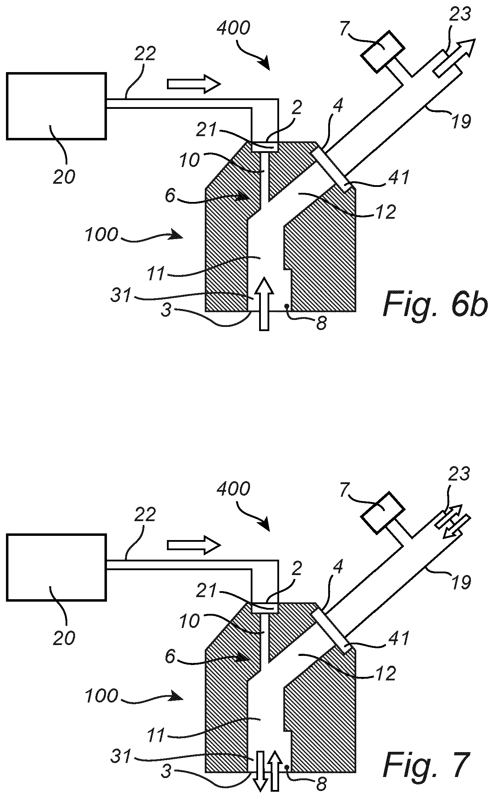

The outlet 4 of the variable flow CPAP generator 6 has an open end 5. In the system 400 shown in Fig. 4, the outlet tube 19 is connected with the open-ended outlet 4 of the device and has an open end 23. If the open end 23 of the outlet tube 19 is occluded, see Figs. 4 and 6a, the pressure delivered to the infant will increase from the pressure set by the variable CPAP generators 6 until the opening pressure of the pressure release valve 7 is reached. In the system 500 comprising the device 300 described with reference to Fig. 3, occlusion of the open end 5 of the outlet 4 of the device 300 will have the same effect. A typical value for the pressure release valve 7 to open is around 20-30 cm H₂O. The increase in pressure results in an inspiratory flow. The pressure in the system 400 will remain at the set positive pressure ventilation pressure until the outlet occlusion is removed. When the occluded open end 5 is opened, see Figure 6b, the pressure delivered to the patient will return to the set CPAP level and this reduction in pressure will lead to an expiratory flow.

During spontaneous breathing, the infant flow and the fresh gas flow leaves the system 400 through the variable flow CPAP generators 6, see Figure 7. This keeps the positive pressure within the airway stable. By varying the driver flow, i.e. the fresh gas flow that generates the CPAP pressure, the CPAP level in the airway can be adjusted as needed.

The patient interface end 3 can be designed in any suitable form such to be suitable for connection with a patient interface. Further, the patient interface can assume a variety of designs suitable for establishing a connection to the patient nasal airways, not shown. Thus, the patient interface can include an opposing pair of nasal prongs, a mask, an endotracheal tube or any other suitable devices.

The system 400, 500 could have a backup system for malfunctioning of the pressure release valve 7. This could either be an alarm, a second release valve or a system that cuts the fresh gas flow.

The pressure measuring device 8 should be positioned as close to the patient as possible to provide accurate recording of the pressure of the gas delivered to the patient. The accuracy will depend on the flow resistance of the patient interface and, for an infant, a low resistance interface should be used if possible. The embodiment of the device described with reference to Figs. 1 and 2 could be regarded as beneficial since there will be less tubes needed in the system 400.

Notably, the device, in accordance with principles of the present disclosure is useful with a wide variety of patient interface configurations that may or may not incorporate some or all of the features described above with respect to the patient interface. Thus, the patient interface is in no way limiting.

Notably, the device, in accordance with principles of the present disclosure is useful with a wide variety of variable flow CPAP generators that may or may not incorporate some or all of the features described above with respect to the variable flow CPAP generators 6. Thus, the model of the variable flow CPAP generators 6 is in no way limiting.

Notably, the device, in accordance with principles of the present disclosure is useful with a wide variety of pressure release valves 7 or similar devices that achieve the purpose of releasing air depending on the pressure in the system. Thus, the model or type of pressure release valve is in no way limiting.

Notably, the device, in accordance with principles of the present disclosure is useful with a wide variety of pressure measuring devices 8 or similar devices that achieve the purpose of measuring the pressure in the system. Thus, the model or type pressure measuring devices is in no way limiting.

A typical CPAP level for resuscitating or stabilising an infant is in the range of 4-10 cm H₂O. A typical peak pressure for PPV is 20-30 cm H₂O. It is obtained by occluding the system and having a correctly adjusted pressure release valve.

Above embodiments of the device and system according to the present disclosure as defined in the appended claims have been described. These should only be seen as merely non-limiting examples.

It is to be noted that for the purposes of his application, and in particular with regard to the appended claims, the word "comprising" does not exclude other elements or steps, and the word "a" or "an" does not exclude a plurality, which per se will be evident to a person skilled in the art.

## Claims

1. A device (100, 200, 300) for positive pressure ventilation (PPV) and continuous positive airway pressure (CPAP) treatment comprising:
- a fresh gas flow inlet (2) arranged to receive a fresh gas flow from a fresh gas flow tube connectable thereto;
- a patient interface end (3) which is connectable with a patient interface;
- an outlet (4) having an open end (5); and
- several variable flow CPAP generators (6),
wherein each of the several variable flow CPAP generators is connected with the fresh gas flow inlet, the patient interface end and the outlet of the device, wherein each of the several variable flow CPAP generators (6) comprises first, second and third connection portions (10, 11, 12), and wherein the first connection portion is connected with the fresh gas flow inlet (2), the second connection portion is connected with the patient interface end (3), and the third connection portion is connected with the outlet (4) of the device.

2. The device (100, 200, 300) according to claim 1, further comprising an inlet chamber (21) arranged at the fresh gas flow inlet (2), a patient interface chamber (31) arranged at the patient interface end (3), and an outlet chamber (4) arranged at the outlet (4) of the device, wherein each of the several variable flow CPAP generators is connected with the fresh gas flow inlet, the patient interface end, and the outlet through the inlet chamber, the patient interface chamber and the outlet chamber, respectively.

3. The device (100, 300) according to any one of claims 1 to 2, wherein the device comprises three variable flow CPAP generators (6).

4. The device (200) according to any one of claims 1 to 2, wherein the device comprises four variable flow CPAP generators (6).

5. The device (300) according to any one of the preceding claims, further comprising a pressure release connection portion (14) having a first end (16) connected with the patient interface end (3) of the device and a second end (17) connectable with a pressure release tube.

6. A system (400, 500) for PPV and CPAP treatment comprising a device (100, 200, 300) according to any one of the preceding claims, a fresh gas flow tube (22), a fresh gas source (20) connected with the fresh gas flow inlet (2) by means of the fresh gas flow tube, and a pressure release valve (7) arranged to prevent an excessive positive pressure in a PPV mode.

7. The system (400, 500) according to claim 6, arranged such that when the open end (5) of the outlet (4) of the device (100, 200, 300) is occluded, the pressure will increase from the variable flow CPAP generators (6) until an opening pressure of the pressure release valve (7) is reached, which increase in pressure results in an inspiratory flow, whereby the pressure in the system will remain at the set PPV pressure until the occlusion is removed from the outlet, and when the occluded outlet is opened, the pressure will return to the set CPAP level, whereby the reduction in pressure leads to an expiratory flow.

8. The system (400, 500) according to claim 6, arranged such that during spontaneous breathing, the patient flow and the fresh gas flow leaves the system through the variable flow CPAP generators (6) keeping the positive pressure within the airway of the patient stable, by varying the flow that generates the CPAP, whereby the CPAP in the airway can be adjusted as needed.

9. The system (400, 500) according to any one of claims 6-8, wherein the pressure release valve is connected with one of the patient interface end (3) and the outlet (4) of the device (100, 200, 300).

10. The system (400, 500) according to any one of claims 6-8 comprising a device (300) according to claim 6, wherein the pressure release valve (7) is connected with the pressure release connection portion (14).

11. The system (400, 500) according to any one of claims 6-10, further comprising a pressure measuring device (8).

12. The system (400, 500) according to claim 11, wherein the pressure measuring device (8) is arranged at the patient interface end (3) of the device (100, 200), or in connection with the pressure release connection portion (14) of the device (300) according to claim 5.

## Patentansprüche

1. Vorrichtung (100, 200, 300) zur Behandlung mit Überdruckbeatmung (PPV *- Positive Pressure Ventilation*) und mit kontinuierlichem positivem Atemwegsdruck (CPAP - *Continuous Positive Airway Pressure*)*,* welche Folgendes umfasst:
- einen Frischgasflusseinlass (2), der zum Empfangen eines Frischgasflusses von einem daran anschließbaren Frischgasflussschlauch geeignet ist;
- ein Patientenschnittstellenende (3), das mit einer Patientenschnittstelle verbunden werden kann;
- einen Auslass (4) mit einem offenen Ende (5); und
- mehrere CPAP-Generatoren mit variablem Durchfluss (6), wobei jeder der mehreren CPAP-Generatoren mit variablem Durchfluss (6) mit dem Frischgasflusseinlass, dem Patientenschnittstellenende und dem Auslass der Vorrichtung verbunden ist, wobei jeder der mehreren CPAP-Generatoren mit variablem Durchfluss (6) einen ersten, einen zweiten und einen dritten Verbindungsabschnitt (10, 11, 12) umfasst, und wobei der erste Verbindungsabschnitt mit dem Frischgasflusseinlass (2) verbunden ist, der zweite Verbindungsabschnitt mit dem Patientenschnittstellenende (3) verbunden ist und der dritte Verbindungsabschnitt mit dem Auslass (4) der Vorrichtung verbunden ist.

2. Vorrichtung (100, 200, 300) nach Anspruch 1, welche ferner eine Einlasskammer (21) angeordnet am Frischgasflusseinlass (2), eine Patientenschnittstellenkammer (31) angeordnet am Patientenschnittstellenende (3) und eine Auslasskammer (41) angeordnet am Auslass (4) der Vorrichtung umfasst, wobei jeder der mehreren CPAP-Generatoren mit variablem Durchfluss durch die Einlasskammer, die Patientenschnittstellenkammer bzw. die Auslasskammer mit dem Frischgasflusseinlass, dem Patientenschnittstellenende und dem Auslass verbunden ist.

3. Vorrichtung (100, 300) nach einem der Ansprüche 1 bis 2, wobei die Vorrichtung drei CPAP-Generatoren mit variablem Durchfluss (6) umfasst.

4. Vorrichtung (200) nach einem der Ansprüche 1 bis 2, wobei die Vorrichtung vier CPAP-Generatoren mit variablem Durchfluss (6) umfasst.

5. Vorrichtung (300) nach einem der vorhergehenden Ansprüche, welche ferner einen Druckentlastungsverbindungsabschnitt (14) mit einem ersten Ende (16), das mit dem Patientenschnittstellenende (3) der Vorrichtung verbunden ist, und einem zweiten Ende (17), das mit einem Druckentlastungsschlauch verbunden werden kann, umfasst.

6. System (400, 500) zur PPV- und CPAP-Behandlung, welches eine Vorrichtung (100, 200, 300) nach einem der vorhergehenden Ansprüche, einen Frischgasflussschlauch (22), eine Frischgasquelle (20), die mithilfe des Frischgasflussschlauchs mit dem Frischgasflusseinlass (2) verbunden ist, und ein Druckentlastungsventil (7), das zum Verhindern eines übermäßigen Überdrucks in einem PPV-Modus geeignet ist, umfasst.

7. System (400, 500) nach Anspruch 6, welches derart angeordnet ist, dass, wenn das offene Ende (5) des Auslasses (4) der Vorrichtung (100, 200, 300) verschlossen ist, der Druck von den CPAP-Generatoren mit variablem Durchfluss (6) ansteigt, bis ein Öffnungsdruck des Druckentlastungsventils (7) erreicht ist, wobei dieser Druckanstieg in einem Einatemfluss resultiert, wodurch der Druck im System auf dem eingestellten PPV-Druck verbleibt, bis der Verschluss vom Auslass entfernt wird, und, wenn der verschlossene Auslass geöffnet wird, der Druck auf das eingestellte CPAP-Niveau zurückkehrt, wodurch die Druckminderung zu einem Ausatemfluss führt.

8. System (400, 500) nach Anspruch 6, welches derart angeordnet ist, dass bei Spontanatmung der Patientenfluss und der Frischgasfluss das System durch die CPAP-Generatoren mit variablem Durchfluss (6) verlassen, wodurch der Überdruck innerhalb der Atemwege des Patienten stabil gehalten wird, indem der Fluss, der den CPAP erzeugt, variiert wird, wodurch der CPAP in den Atemwegen nach Bedarf angepasst werden kann.

9. System (400, 500) nach einem der Ansprüche 6-8, wobei das Druckentlastungsventil entweder mit dem Patientenschnittstellenende (3) oder dem Auslass (4) der Vorrichtung (100, 200, 300) verbunden ist.

10. System (400, 500) nach einem der Ansprüche 6-8, welches eine Vorrichtung (300) nach Anspruch 6 umfasst, wobei das Druckentlastungsventil (7) mit dem Druckentlastungsverbindungsabschnitt (14) verbunden ist.

11. System (400, 500) nach einem der Ansprüche 6-10, welches ferner eine Druckmessvorrichtung (8) umfasst.

12. System (400, 500) nach Anspruch 11, wobei die Druckmessvorrichtung (8) am Patientenschnittstellenende (3) der Vorrichtung (100, 200) oder in Verbindung mit dem Druckentlastungsverbindungsabschnitt (14) der Vorrichtung (300) nach Anspruch 5 angeordnet ist.

## Revendications

1. Dispositif (100, 200, 300) pour un traitement par ventilation en pression positive (PPV) et par ventilation en pression positive continue (CPAP) comprenant :
- une entrée de circulation de gaz neuf (2) agencée pour recevoir une circulation de gaz neuf d'un tube de circulation de gaz neuf pouvant être connecté dessus ;
- une extrémité d'interface patient (3) agencée pour être connectée à une interface patient ;
- une sortie (4) ayant une extrémité ouverte (5) ;
- plusieurs générateurs de CPAP à circulation variable (6),
- dans lequel chacun des plusieurs générateurs de CPAP à circulation variable (6) est connecté à l'entrée de circulation de gaz neuf, l'extrémité d'interface patient et la sortie du dispositif, dans lequel chacun plusieurs des générateurs de CPAP à circulation variable (6) comprend des première, deuxième et troisième parties de connexion (10, 11, 12), et dans lequel la première partie de connexion est connectée à l'entrée de circulation de gaz neuf (2), la deuxième partie de connexion est connectée à l'extrémité d'interface patient (3), et la troisième partie de connexion est connectée à la sortie (4) du dispositif.

2. Dispositif (100, 200, 300) selon la revendication 1, comprenant en outre une chambre d'entrée (21) agencée sur l'entrée de circulation de gaz neuf (2), une chambre d'interface patient (31) agencée sur l'extrémité d'interface patient (3) et une chambre de sortie (4) agencée sur la sortie (4) du dispositif, dans lequel chacun des plusieurs générateurs de CPAP à circulation variable est connecté à l'entrée de circulation de gaz neuf, l'extrémité d'interface patient et la sortie par la chambre d'entrée, la chambre d'interface patient et la chambre de sortie, respectivement.

3. Dispositif (100, 300) selon l'une quelconque des revendications 1 à 2, dans lequel le dispositif comprend trois générateurs de CPAP à circulation variable (6).

4. Dispositif (200) selon l'une quelconque des revendications 1 à 2, dans lequel le dispositif comprend quatre générateurs de CPAP à circulation variable (6).

5. Dispositif (100, 200, 250) selon l'une quelconque des revendications précédentes, comprenant en outre une partie de connexion de libération de pression (14), ayant une première extrémité (16) connectée à l'extrémité d'interface patient (3) du dispositif et une deuxième extrémité (17) pouvant être connectée à un tube de libération de pression.

6. Système (400, 500) pour le traitement par PPV et CPAP comprenant un dispositif (100, 200, 300) selon l'une quelconque des revendications précédentes, un tube de circulation de gaz neuf (22), une source de gaz neuf (20) connectée à l'entrée de circulation de gaz neuf (2) au moyen du tube de circulation de gaz neuf, et une soupape de libération de pression (7) agencée pour empêcher une pression positive en excès dans un mode de PPV.

7. Système (400, 500) selon la revendication 6, agencé de telle façon que lorsque l'extrémité ouverte (5) de la sortie (4) du dispositif (100, 200, 300) est fermée, la pression augmentera des générateurs de CPAP à circulation variable (6) jusqu'à ce qu'une pression d'ouverture de la soupape de libération de pression (7) soit atteinte, laquelle augmentation de pression résulte en un flux inspiratoire, ce par quoi la pression dans le système restera à la pression de PPV réglée jusqu'à ce que l'occlusion soit retirée de la sortie, et lorsque la sortie occluse sera ouverte, la pression retournera au niveau de CPAP réglé, ce par quoi la réduction de pression conduit à un flux expiratoire.

8. Système (400, 500) selon la revendication 6, agencé de telle façon que pendant la respiration spontanée, le débit du patient et la circulation de gaz neuf quittent le système à travers les générateurs de CPAP à circulation variable (6), maintenant la pression positive à l'intérieur de la ventilation du patient stable, en faisant varier la circulation qui génère la CPAP, ce par quoi la CPAP dans la ventilation peut être ajustée selon les besoins.

9. Système (400, 500) selon l'une quelconque des revendications 6-8, dans lequel la soupape de libération de pression est connectée à l'une de l'extrémité d'interface patient (3) et de la sortie (4) du dispositif (100, 200, 300).

10. Système (400, 500) selon l'une quelconque des revendications 6-8, comprenant un dispositif (300) selon la revendication 6, dans lequel la soupape de libération de pression (7) est connectée à la partie de connexion de libération de pression (14).

11. Système (400, 500) selon l'une quelconque des revendications 6-10, comprenant en outre un dispositif de mesure de pression (8).

12. Système (400, 500) selon la revendication 11, dans lequel le dispositif de mesure de pression (8) est agencé sur l'extrémité d'interface patient (3) du dispositif (100, 200) ou en connexion avec la partie de connexion de libération de pression (14) du dispositif (300) selon la revendication 5.
